# EUROPEAN PATENT SPECIFICATION

(11) **EP 3 363 450 B1**
(45) Date of publication and mention of the grant of the patent: **22.09.2021**
(21) Application number: 16854830.3
(22) Date of filing: 19.08.2016
(51) Int. Cl.: A61K 36/9066, A61P 17/06, A61K 36/896, A61K 36/65, A61K 36/736, A61K 36/67

(54) **TRADITIONAL CHINESE MEDICINE COMPOSITION FOR TREATING PSORIASIS**
TRADITIONELLE CHINESISCHE MEDIZINISCHE ZUSAMMENSETZUNG ZUR BEHANDLUNG VON PSORIASIS
COMPOSITION DE MÉDECINE TRADITIONNELLE CHINOISE POUR LE TRAITEMENT DU PSORIASIS

(30) Priority: 15.10.2015 CN 201510670944
(43) Date of publication of application: 22.08.2018
(73) Proprietor: Guangdong Provincial Hospital Of Chinese Medicine, Guangzhou, Guangdong 510120 (CN)
(72) Inventor: LU, Chuanjian, Guangzhou Guangdong 510120 (CN); ZHAO, Ruizhi, Guangzhou Guangdong 510120 (CN); XUAN, Guowei, Guangzhou Guangdong 510120 (CN); HAN, Ling, Guangzhou Guangdong 510120 (CN); CHEN, Gengxin, Guangzhou Guangdong 510120 (CN); YAN, Yuhong, Guangzhou Guangdong 510120 (CN); LIU, Lijuan, Guangzhou Guangdong 510120 (CN)
(74) Representative: Bird & Bird LLP
(86) International application number: PCT/CN2016/095963
(87) International publication number: WO 2017/063448

(56) References cited:
- CN-A- 101 632 827
- CN-A- 103 446 525
- CN-A- 105 213 971
- US-A- 5 997 875
- DATABASE WPI Week 201553 Thomson Scientific, London, GB; AN 2015-391438 XP002789372, & CN 104 547 787 A (SHANDONG JIATE PHARM TECHNOLOGY CO LTD) 29 April 2015 (2015-04-29)
- DATABASE WPI Week 201546 Thomson Scientific, London, GB; AN 2015-383110 XP002789373, & CN 104 524 366 A (LIU J) 22 April 2015 (2015-04-22)
- DATABASE WPI Week 201501 Thomson Scientific, London, GB; AN 2014-W62058 XP002789374, & CN 104 056 139 A (CONG D) 24 September 2014 (2014-09-24)
- DATABASE WPI Week 201423 Thomson Scientific, London, GB; AN 2014-B16938 XP002789375, & CN 103 386 051 A (YU Q) 13 November 2013 (2013-11-13)
- DATABASE WPI Week 201023 Thomson Scientific, London, GB; AN 2010-B59692 XP002789376, & CN 101 637 585 A (LU S) 3 February 2010 (2010-02-03)
- DATABASE WPI Week 201545 Thomson Scientific, London, GB; AN 2015-36393S XP002789377, & CN 104 510 950 A (ZHANG X) 15 April 2015 (2015-04-15)
- DATABASE WPI Week 201401 Thomson Scientific, London, GB; AN 2013-S00702 XP002789378, & CN 103 142 903 A (QINGDAO MUNICIPAL HOSPITAL) 12 June 2013 (2013-06-12)
- DATABASE WPI Week 201109 Thomson Scientific, London, GB; AN 2010-P14485 XP002789379, & CN 101 856 455 A (BOHAI SHIPBUILDING HEAVY IND CO LTD) 13 October 2010 (2010-10-13)
- DATABASE WPI Week 201437 Thomson Scientific, London, GB; AN 2014-J35529 XP002789380, & CN 103 656 369 A (QINGDAO MEDICAL DISINFECTION TECH CENT) 26 March 2014 (2014-03-26)
- DATABASE WPI Week 201368 Thomson Scientific, London, GB; AN 2013-P54313 XP002789381, & CN 103 041 237 A (WU D) 17 April 2013 (2013-04-17)
- DATABASE WPI Week 201322 Thomson Scientific, London, GB; AN 2013-D16890 XP002789382, & CN 102 784 296 A (XU H) 21 November 2012 (2012-11-21)
- DATABASE WPI Week 201017 Thomson Scientific, London, GB; AN 2010-B49890 XP002789383, & CN 101 632 827 A (UNIV NO 2 CLINICAL MEDICINE COLLEGE GUAN) 27 January 2010 (2010-01-27)
- DATABASE WPI Week 201611 Thomson Scientific, London, GB; AN 2015-69651S XP002789384, & CN 104 971 312 A (LI Q) 14 October 2015 (2015-10-14)
- LI, TAO;: 'Experience in Treating Psoriasis' HEILONGJIANG SCIENCE AND TECHNOLOGY INFORMATION vol. 19, 31 December 2007, page 219, XP009509395

## Description

### Background of the Present Invention

### Field of Invention

The present invention relates to a traditional Chinese medicine composition, and the use of the traditional Chinese medicine composition for treating skin diseases.

### Description of Related Arts

Psoriasis is a genetically predisposed, systemic, chronic, inflammatory skin disease. Its typical features are recurrent scaly erythema or papules with itching or pain, and a thin film phenomenon and punctate hemorrhage. Nails and joints will be involved as well. Its pathogenesis is not yet fully clear, mainly concerned with epidermal keratinocytes and immune dysfunction, related to complex immune and inflammatory response. At present, there are 125 million psoriasis patients throughout the world, the morbidity of psoriasis accounts for 3% of the world's population, and trends to raise year by year. Globally, the highest morbidity of psoriasis is 8.2% of Norway. Total morbidity is 0.72% in China, there has been 10 million patients at present. According to the results comparing data of two large psoriasis epidemiological survey conducted in 1984 and 2010, the morbidity of psoriasis is increasing in China. Psoriasis vulgaris accounts for 85-90%, psoriasis can be divided into early onset and late onset according to the age of onset, the morbidity of early onset psoriasis is 4.5 times higher than the morbidity of late onset psoriasis in China.

Psoriasis appearance does not look good, resulting in patients having inferiority tendencies, and also affecting their employments and quality of life. Long-term treatment costs not only bring severe economic and mental burdens to patients, and high morbidity and long-term treatment also cause a huge social burden.

The medicaments used are determined according to the severity of the disease in traditional treatment of psoriasis, local treatment is mainly used for mild patients, systemic treatment is used for moderate and severe patients, wherein the local treatment refers to local application of corticosteroids, tar, anthralin, vitamin D analogues, tazarotene and so on. Their efficacy and side effects show in Table 1:

**Table 1 Advantages and disadvantages of currently common topical drugs for psoriasis**

| Drugs | Advantages | Disadvantages |
|---|---|---|
| Vitamin analogues | Effective | Easy to relapse, irritating, hypercalcemia |
| Corticosteroids | Effective | Easy to relapse, skin atrophy, bone marrow suppression, alopecia, teratogenesis |
| Anthralin | Effective | Dithranol dyeing skin, skin irritation, contaminating clothing |
| Tar | Effective | Slow onset, skin irritation, foreign odor, folliculitis, possibly carcinogenic |
| Retinol | Effective | Irritation, teratogenesis |
| Tacrolimus | Only effective for thin skin lesions | Protection from light, instantaneous burning sensation |

Generally, for the moderate and severe patients, the systemic treatment is used additively on the basis of the use of the local treatment. The first-line drugs are mainly methotrexate and cyclosporine, vitamin A (retinol) and its derivatives, fumarate lipid, acitretin and so on, their efficacy and side effects show in Table 2.

**Table 2 Advantages and disadvantages of currently common systemic treatment drugs for psoriasis**

| Drugs | Advantages | Disadvantages |
|---|---|---|
| Methotrexate | Effective, the efficient is 20-40% | Nausea, myelosuppression, hepatotoxicity, pulmonary fibrosis, stomatitis, mucosal ulcers |
| Cyclosporine | Rapid onset, the efficient is 50-70% | Myelosuppression, renal injury, hypertension, hirsutism, Hyperuricemia, hypomagnesemia |
| Vitamin A | Effective, the efficient is 25% | Teratogenesis, hyperlipidemia, elevated liver enzymes |
| Fumarate lipid | Effective, the efficient is 50-70% | Nausea, abdominal pain, diarrhea (occasionally), lymphopenia, abnormal liver function |
| Acitretin | Effective, not suitable for women in reproductive years | Slow onset, teratogenesis, hyperlipidemia, liver dysfunction, alopecia, dry skin and mucous membranes, myalgia, sweating |
| Hydroxyurea | Effective | Myelosuppression |

According to the survey of the European Psoriasis Foundation, only about 26% of patients are satisfied with the traditional treatment, it has been found in a British survey that 44% of patients prefer the systemic treatment over the local administration. The European Psoriasis Association's survey of 17,994 patients showed that only 27% of patients were satisfied with the conventional treatment, and that unsatisfactory items included serious side effects, time consuming and low effectiveness of treatment. Therefore, the development of safe and effective drugs for treating psoriasis is still a hotspot in international medicine research and development.

In the earlier stage, the applicant has found a traditional Chinese medicine compound for the treating psoriasis in clinical practice, has applied for and has been granted a patent right (patent no. ZL200910091066.5). Because of comprising many herbs, the quality control is difficult. After several years of exploration, the applicant has found that it can also play a role in the treatment of psoriasis when reducing herbs and increasing dosage at same time, therefore, applied for the second patent (201210183904.3). However, the toxicity of drugs is also increased due to increasing dosage.
CN 104547787 A realtes to a traditional Chinese medicine preparation comprising the following active ingredients in parts by weight: 30-60 parts of rhizoma smilacis glabrae, 20-40 parts of saffron crocus, 30-60 parts of herba cistanches, 10-20 parts of periostracum cicada, 20-40 parts of nodus nelumbinis rhizomatis, 30-60 parts of realgar, 10-20 parts of radix paeoniae rubra and 10-20 parts of palembus dermestoides.
CN104524366 A discloses a traditional Chinese medicine composition comprising the following components by weight: 25-35g of folium ilicis purpurea, 15-25g of chinaroot greenbrier, 10-20g of dried radix rehmanniae and 5-15g of liquorice.
CN 104056139 A relates to a traditional Chinese medicine composition useful to treat psoriasis, comprising chinaroot greenbrier (10-19 wt/%), smoked plum (9-13 wt%) and sarcandra (7-9 wt%).
CN 103386051 A discloses a traditional Chinese medicine composition for curing psoriasis, comprising also rhizoma smilacis glabrae (4-5 pts. wt.) and (4-7 pts. wt.) root of common peony.
CN 101637585 A describes a Chinese herbal preparation for treating posirasis, which adopts 58 raw medicinal materials such as astragalus root, suberect spatholobus stem, Job stears, glabrous greenbrierrhizome, spreading hedyotis herb, barbed skullcap herb, motherwort herb and the like.
CN 104510950 A discloses a traditional Chinese medicine to be used for treating intractable psoriasis, including blackfruit greenbrier rhizome and leaf (2-4 pts. wt.)
CN 103142903 A relates to a traditional Chinese medicine useful for treating psoriasis, containing rhizoma smilacis glabrae (6-20 pts. wt.) and root of common peony (1-12 pts. wt.).
CN 101856455 A is directed to a traditional Chinese medicine useful for treating psoriasis, containing red peony and rhizome smilacis glabrae.
CN 103656369 A describes a traditional Chinese medicine useful for preventing psoriasis, containing rhizoma smilacis glabrae (3-9 wt%) and radix paeoniae rubra (6-9 wt%).
CN 103041237 A is directed to a Chinese medicinal mixture for treating psoriasis, being mainly prepared from the following components by weight: 15g of raw rehmannia, 10g of red peony root, 12g of peony tree bark, 10g of lithospermum, 12g of honeysuckle, 25g of glabrous greenbrier rhizome, 15g of snake slough, 8g of golden thread, 6g of charred schizonepeta, 25g of raw gyp, 12g of rhizoma anemarrhenae, and 5g of raw liquorice.
CN 102784296 A reveals a traditional Chinese medicine for treating psoriasis, comprising fructus mume (20-25 g) and radix paeonia alba (10-15g).
CN 104971312 A relates to a medicinal oil used for treating psoriasis, including curcuma zedoary (8-13 pts. wt.) and smoked plum (12-17 pts. wt.).

In US 5,997,875 A a herbal treatment composition is provided which includes Sarcandra glabra and a polysaccharide derived from medicinal mushrooms. Methods for treating psoriasis and purpura using the composition are also disclosed.

### Summary of the Present Invention

An object of the present invention is to disclose a traditional Chinese medicine composition for treating psoriasis, to overcome the disadvantages existing in the prior art and meet people's needs.

The traditional Chinese medicine composition for treating psoriasis of the present invention is prepared from materials in the following parts by weight:
5-15 parts of paeoniae radix rubra, 3-9 parts of curcumae rhizoma, 10-20 parts of sarcandrae herba (Sarcandra glabra), 10-20 parts of smilacis glabrae rhizoma, 5-15 parts of mume fructus;
preferably, it is prepared from materials in the following parts by weight:
9 parts of Red peony root(paeoniae radix rubra), 6 parts of curcumae rhizoma (rhizoma zedoariae), 15 parts of sarcandrae herba (sarcandra glabra (Glabrous Sarcandra Herb), 15 parts of smilacis glabrae rhizoma (glabrous greenbrier rhizome), 6 parts of mume fructus (black plum).

Preparation method:
The above-mentioned medicinal materials were soaked for 1h with 10 times weight of water, extracted 2 times (each 45 min), filtrated, the extract was filtrated and concentrated, when the relative density up to 1.10-1.20 measured at 50-60 °C , the extract was cooled to room temperature, added ethanol to reach an alcohol content of 70%, standing for 24 h, filtrated, after removing the ethanol, the extract was concentrated until the relative density was 1.30-1.40, the traditional Chinese medicine composition is obtained.

The in vitro test and clinical data show that the traditional Chinese medicine composition has a remarkable therapeutic effect on psoriasis, psoriasis arthritis, rheumatoid arthritis or malignant tumor, and can be used for preparation of medicaments for treating psoriasis, psoriasis arthritis, rheumatoid arthritis or malignant tumor, and has less toxicity.

In the present invention, the medicaments are administered to patients in need of treatment by oral administration, injection administration, sublingual administration, rectal administration, vaginal administration, transdermal administration, and spray inhalation route at a dosage of 26 to 78 grams per day, which is adjusted by the physician according to the condition; wherein, the dosage is based on medicinal materials.

The present invention also refers to a traditional Chinese medicine formulation comprising a therapeutically effective amount of the traditional Chinese medicine composition and a pharmaceutically acceptable carrier. The carrier comprises excipients such as starch, dextrin, sodium carboxymethyl starch , carboxymethyl cellulose, carboxypropyl cellulose, polyethylene glycol, glycerin, sorbitol, powdered sugar, wine, vinegar, lactose, compressible starch, microcrystalline cellulose, inorganic salts, mannitol, propylene glycol, water, surfactant, benzoic acid, sorbic acid, nipagin, sodium alginate, arabic gum, gelatin, methylcellulose, sodium carboxymethylcellulose, sodium salicylate, sodium chloride, and so on, sweetening agents such as stevioside, sucrose, aspartame, monosaccharides, saccharin sodium, lactose, mannitol, sorbitol, acesulfame and so on.

Preferably, the traditional Chinese medicine formulation is in the form of tablets, granules, capsules, pills, dropping pills, powders, electuary, syrups, emplastrum, mixtures, medicated liquor, tinctures, pastille, liquid extract and extract, plasters, gels, ointments, tea agents, lotions, coating agents, liniments, aerosols or sprays.

Preparation method of the traditional Chinese medicine formulation is conventional. The traditional Chinese medicine formulation is mixed with a carrier and then prepared into tablets, granules, capsules, pills, dropping pills, powders, electuary, syrups, emplastrum, mixtures, medicated liquor, tinctures, lozenges, liquid extract and extract, plasters, gels, ointments, tea agents, lotions, coating agents, liniments, aerosols or sprays.

The present invention has the advantages that:
the therapeutic effect can be achieved by a smaller dosage. Compared with the patent of 200910091966.5, the present invention has the advantages of reducing herbs and enhancing the curative effect; comparing with the patent of 201210183904.3, the present invention has the advantages of playing better curative effect by a small dosage and having significantly reduced toxicity; comparing with the two patents, the present invention has the advantages that the onset is rapid, and the recurrence can be delayed. The effect is more prominent for psoriasis vulgaris (PASI50 up to 60%), especially for early onset patients. The incidence of complications such as cardiovascular events, psoriasis arthritis, diabetes and so on can be reduced (complication rate of the patients of early drug treatment is reduced by 20%), the quality of life can be significantly improved, and the effect is better.

### Detailed Description of the Preferred Embodiments

### EXAMPLE 1

### Formula 1

Paeoniae radix rubra (Red peony root) 9g, curcumae rhizoma (rhizoma zedoariae) 6g, sarcandrae herba (sarcandra glabra, Glabrous Sarcandra Herb) 15g, smilacis glabrae rhizome (glabrous greenbrier rhizome) 15g, mume fructus (black plum) 6g.

Preparation method:
The above-mentioned medicinal materials were soaked for 0.5h with 10 times weight of water, extracted 2 times, the extract was filtrated, the filtrate was concentrated to a relative density of 1.07-1.09, spray-dried, and the dried powder was evenly mixed with starch and magnesium stearate, and tabletting to obtain the product.

### EXAMPLE 2

### Formula 2

Paeoniae radix rubra (Red peony root) 15g, curcumae rhizoma (rhizoma zedoariae) 3g, sarcandrae herba (sarcandra glabra, Glabrous Sarcandra Herb) 20g, smilacis glabrae rhizome (glabrous greenbrier rhizome) 10g, mume fructus (black plum) 15g.

The above-mentioned medicinal materials were soaked for 0.5h with 70% ethanol, reflux extracted 2 times (each 0.5h), filtrated, the filtrate was concentrated to a relative density of 1.13-1.15, adding sucrose powder, granulated, dried, sphericized and prepared into granule.

### EXAMPLE 3 Clinical study results

### 1.1 Diagnostic criteria

Western medicine diagnostic criteria for psoriasis vulgaris: (psoriasis treatment guidelines 2008 Edition - recommended by American Academy of Dermatology; Yang Guoliang, Wang Xiansheng. Modern Dermatology. Shanghai: Shanghai Medical University Press, 2005)

At the beginning of psoriasis vulgaris, the basic damage is red papule or rash having a size from millet to mung bean, covered with silvery-white scales, scales are less in acute damage than in chronic damage. After scraping the scales, a bright red film appearing, which is known as the thin film phenomenon. Scattered small bleeding points in dew-shape can appear after gently scratching the thin film (Auspitz phenomenon). The basic damage appears mostly in the scalp, elbow, knee, joint extensor and sacral. The damage also appears point-like depression of the deck and oil droplets below the deck. Red patches having a clear edge appear in mucous membrane, which has no scales. There are silvery-white scales on oral lips. There are grayish yellow or white ring patches on buccal mucosa and palate. Eye lesions are rarely occurred, such as blepharitis, conjunctivitis.

The stage of psoriasis vulgaris: (Yang Guoliang, Wang Xiansheng. Modern Dermatology. Shanghai: Shanghai Medical University Press, 2005)
Progressive stage: acute attack phase, and having isomorphic reactions at this stage.
Stable stage: inflammation regression, no kainogenesis of skin lesions, being in a quiescent condition.
Declining stage: skin lesions thinning, red fading, until the skin lesions disappearing, leaving hypopigmented or pigmentation spots.

Early-onset, late-onset diagnostic criteria:
Early onset (type I) patients have an onset before 40 years old, which is associated with human leukocyte antigen (HLA), especially HLA-Cw6, B57, DR7, peak of incidence is about 20 years old; late onset (type II) patients have an onset after 40 years old, do not express HLA-DR7 and overexpress HLA-Cw2.

Standard of differentiation of symptoms and signs in the traditional Chinese medicine: the differentiation of symptoms and signs is performed with reference to "Standard for Traditional Chinese Medicine Practitioners in the People's Republic of China", "Guidelines for Clinical Research of New Drugs of Traditional Chinese Medicine" on the criteria for TCM Syndrome of psoriasis.

### 1.2 Inclusion criteria

(1) Psoriasis patients of stable stage, moderate, plaque, duration>1 year.
(2) Patients of 18-65 years old.
(3) Patients having the degree of skin lesions of 3 <PASI≤10, and BSA≤10%.
(4) Patients signed informed consent.

### 1.3 Exclusion criteria

(1) Patients who mainly have guttate skin lesions, or who skin lesions alone appear in the face, scalp, nails, wrinkles, glans, mucosa, palmoplantar parts.
(2) Women who are in pregnancy, lactation or planning pregnancy within 1 year.
(3) Patients having psychological measurement scale SAS standard score> 50 or SDS standard score> 53, or combined with other mental diseases.
(4) Patients combined with serious primary diseases of circulatory system, respiratory system, digestive system, urinary system, endocrine system and hematopoietic system, and so on, which unable to be controlled by conventional medications, patients combined with tumor, patients with a serious infection, and balance disorder of water, electrolyte and acid-base, and patients combined with calcium-metabolism dysregulation.
(5) Patients who are known to be allergic to drugs used in this study.
(6) Person who is participating in other drug clinical trial or participated in other clinical trials within 1 month.
(7) Person who had been treated with topical drugs such as hormones, retinoids, or ultraviolet light within 2 weeks; person who had received systemic treatment within 4 weeks; person who had been treated with biological agents within 12 weeks.
(8) Patients who have acute psoriasis progression and red skin disease tendencies.
(9) Patients who need to carry out a western medicine systemic treatment.

### 1.4 Criteria of removing patients

(1) Those who do not meet the criteria of entry after enrollment.
(2) Those who have no available data after enrollment.

### 2. Grouping and course of treatment

### 2.1 Grouping

The qualified psoriasis vulgaris patients were randomly divided into the experimental group of 2000910091066.5, the experimental group of 201210183904.3 and the group of the present invention. The qualified patients were allocated to each group at a ratio of 1: 1: 1 by a way of random distribution method.

### 2.2 Course of treatment

With reference to the domestic and foreign literatures and combining with the results of the previous study, the introduction period was 2 weeks, the treatment period was 12 weeks and the follow-up period was 12 weeks.

### 3. Observation indexes and observation time points

### 3.1 Baseline information

(1) Demographic indexes: date of birth, age, sex, ethnic groups, nationality, marital status, education, height, weight, occupation, long-term residence area, and so on.
(2) Vital signs: body temperature, respiration, heart rate, blood pressure.
(3) History of allergy: history of drug, food or contact allergy.
(4) Disease and treatment history: psoriasis onset, family history, history of past treatment and other diseases and treatment history.
(5) Symptoms of traditional Chinese medicine, tongue and pulse condition.
(6) Stage of psoriasis vulgaris disease: progressive stage, stable stage, declining stage.
(7) Auxiliary examination: blood routine, urine routine, liver function, renal function, biochemistry, blood calcium, erythrocyte sedimentation rate, C-reactive protein, coagulation, blood lipid, electrocardiogram and system biology index; immunological index.
(8) PASI, BSA, VAS, DLQI scale score.

### 3.2 Clinical observation indexes and observation time points

(1) PASI score, BSA, VAS: recorded every 2 weeks during the treatment period and every 2 weeks during the follow-up period. During all observations and follow-up period, those who had condition aggravation should be visited and record at any time.
(2) DLQI: during all observations and follow-up period, recorded every 4 weeks.
(3) Photography of skin lesions: the target skin lesion area and severity and the tongue condition were record by the doctor using the chromatic aberration corrected digital imaging equipment in each visit.
(4) Drug combination: all of the drug combinations of patients were recorded during study period, and use of external drugs and emergency drugs were also recorded. The causes, drug name, dosage and duration of the drug combination were recorded.
(5) Adverse events and adverse reactions: All adverse events and adverse reactions occurred during the study period were recorded and the treatment procedure was recorded.
(6) Drug delivery and recovery: the number of drugs dispensed per visit was recorded, and the number of drugs remaining in the last visit was recorded.

### 3.3 Main outcome measures:

(1) PASI-50
   Percentage of patients with psoriasis skin lesion area and severity index (PASI) decreased by 50% before and after an intervention, wherein which was recorded every 2 weeks during the treatment period and every 2 weeks during follow-up period. The target skin lesion area and severity were record by the doctor using the chromatic aberration corrected digital imaging equipment, and were compared with a standard palette of the psoriasis patch color and infiltration degree introduced from Japan, to evaluate PASI objectively.
(2) The relapse rate of psoriasis during the treatment and follow-up periods
   The relapse is defined as a case that skin lesions are reappeared or exacerbated in patients after improvement (reaching to PASI-50) and PASI total score is increased to reach 50% of baseline [Recommended Standard of American Dermatology Association Expert Group, Gordon KB, Feldman SR, Koo JYM, et al. Definitions of Measures of Effect Duration for Psoriasis Treatments. Arch Dermatol, 2005, 141].

### 3.4 Secondary outcome measures:

(1) Improvement rate of PASI score:
   Improvement rate of PASI score = (PASI score before the intervention - PASI score after the intervention) / PASI score before the intervention× 100%, PASI score was observed and recorded as described above.
(2) PASI-75: Percentage of patients with psoriasis skin lesion area and severity index (PASI) decreased by 75% before and after an intervention, PASI score was observed and recorded as described above.
(3) Skin lesion body surface area (BSA): which was evaluated before the intervention, during the observation period and during the follow-up period, wherein recorded every week during the treatment period and every 2 weeks during the follow-up period.
(4) Time of onset: time of onset is the time that PASI-50 was achieved for the first time in patients during the observation period, that is the time interval from the beginning of treatment to PASI score improvement of 50%. If PASI improvement of 50% has not been achieved during the patient's observation period, considered invalid.
(5) Rebound rate: rebound is as a case that skin lesions are reappeared or exacerbated in patients after improvement (reaching to PASI-50) and PASI total score is increased to reach 125% of baseline. Or transforming into the pustular psoriasis or erythroderma psoriasis.

### 4. Safety Observation and Evaluation

Laboratory safety testing items include blood routine, urine routine, biochemistry, erythrocyte sedimentation rate, C-reactive protein, coagulation, blood lipid and electrocardiogram (which were recorded at week 0 and week 12), system biology index (which was recorded at week 0 and week 12, and recorded when PASI-50 and PASI-75 were reached and recurrence and rebound were appeared in patients). Abnormal changes in laboratory results should be noted, if necessary, its relevance with studying drugs needs to be reviewed and further determined and evaluated.

Serious adverse events or serious adverse reactions should be promptly reported to the person in charge of the research site, the person in charge of the task group and the contact person. All adverse events were analyzed and evaluated for association with the studying drugs.

### 4.1 Definition of adverse events

Adverse events refer to any adverse medical events that occur in subjects in this clinical study, regardless of whether the events are causally related to the studying drugs used above.

In clinical studies, investigators should still record all adverse events observed, resulting from noninduction questioning, or complained by subjects, regardless of which group occur the adverse events and whether the adverse event is associated with the treatment regimen. At the same time, newly occurring diseases or original condition aggravations should be reported during the study period. Poor clinical interventions result should not be recorded as adverse events.

A total of 90 patients were enrolled in this study, 30 patients in each group, the administration method is as follows:
The dosage was 52 g / day, and the dosage was based on medicinal materials.

The above-mentioned medicinal materials were extracted according to example 1, and prepared into 0.3g / ml standard decoction of crude drug, which was orally administered twice a day.

The results are shown in Table 3.

**Table 3 The clinical effect of the drugs of the present invention in the treatment of psoriasis**

| Observation targets | Group of patent 200910091066.5 | Group of patent 201210 183904.3 | Group of the present invention |
|---|---|---|---|
| PASI 50 | 50% | 45% | 60% |
| Relapse rate | 30% | 32% | 18% |
| PASI 75 | 30% | 25% | 40% |
| Improvement rate of PASI score | 68% | 60% | 80% |
| Improvement rate of skin lesion body surface area | 65% | 63% | 78% |
| Time of onset | 4 weeks | 4 weeks | 3 weeks |
| Rebound rate | 20% | 25% | 10% |

Three formulations had no severe toxicity during the administration.

### EXAMPLE 4

Effects of the medicaments of the present invention on the mouse tail scaled epidermis:
60 KM mice weighing 18-25g with half males and half females were selected in this study, the mice were qualified in an adaptive breeding observation. The mice were layered with Excel according to weights, 6 mice every layer, and the mice in each layer were randomly assigned to each group, 10 mice every group, and the mice were divided into 6 groups in total, that is normal control group, the group of the present invention, the group of patent 200910091066.5, the group of the patent of 201210183904.3, the group of methotrexate tablet and the group of yujin yinxie tablet. The mice in each group were given by intragastric administration at a volume of 20 mL·kg^{- 1}·d⁻¹ in the morning, once every day for 14 days. The normal control group was given equal volume of pure water. The mice were sacrificed one hour after the last administration, and a long strip of the dorsal skin of the root of the mouse tail (about 1.0 cm x 0.2 cm) was took off and fixed with 10% formaldehyde solution, embedded in paraffin, and stained with HE. The tail scales of each mouse were observed under an optical microscope. Those where the granulosa layers arranged in rows are in the epidermis of scales between the two follicular orifice, are known as the scales with granulosa layer. The number of scales possessing the granulosa layer was calculated for each 100 scales, the results were expressed as a percentage.

Results: The epithelial granulosa cells of the tail scales of the mice were naturally absent, and only a few granulosa cells were found in the hair follicles, so it could be simulated as the pathological feature of psoriasis parakeratosis. Therefore, mouse tail epidermal model is a natural model, is one of currently recognized experimental pathological models of psoriasis. Histopathological examination showed that the epidermal granulosa cells in the normal control group were less than those in the other groups, and the drug groups showed epithelial changes such as granulosa layer hyperplasia.

The results of the data showed that the group of present invention, the group of patent 201210183904.3, the group of patent 2009100091066.5 can significantly promote the formation of the epidermis granulosa layer of the mouse tail scale, and the difference was significant (P <0.05 or *P* <0.01) compared with the normal control group; the group of methotrexate tablet and the group of yujin yinxie tablet can significantly promote the formation of the epidermis granulosa layer of the mouse tail scale, and the difference was significant *(P* <0.05 or *P* <0.01) compared with the normal control group. The results are shown in Table 4.

**Table 4 Effect of the drugs of the present invention on the formation of the epidermis granulosa layer of the mouse tail scale (x̅ ± s , n = 10)**

| Group | Dosage (g crude drug·kg^{- 1}·d⁻¹) | The number of scales in the granulosa layer |
|---|---|---|
| Normal control group | | 30.25± 6.64 |
| Group of the present invention | 10.51 | 38.21±5.45^{∗∗} |
| Group of 201210183904.3 | 21.62 | 45.10±7.12^{∗∗} |
| Group of 2009100091066.5 | 25.22 | 38.38±9.50^{∗} |
| Group of methotrexate tablet | 3.06 mg | 43.65±11.18^{∗∗} |
| Group of yujin yinxie tablet | 1.30 | 39.76±7.46^{∗} |

| | | |
|---|---|---|
| Note: Compared with the normal control group, *^{∗}P>* 0.05, *^{∗∗}P* <0.01. | | |

The results showed there is no significant difference between the group of the present invention and the positive control drugs. The effect of the present invention was equal to or slightly superior to that of the previous two patents, but the dosage was greatly reduced.

### EXAMPLE 5

Effects of the drugs of the present invention on propranolol-induced guinea pig psoriasis model:
The preparation of 5% propranolol hydrochloride emulsion: 5g propranolol hydrochloride was dissolved with 50% ethanol, 5 mL 1,2-propanediol was added as a composite accelerator, and 5 g PVPK30 was added as a film-forming material. 50% ethanol was added to 100mL, and preparing into 5% propranolol hydrochloride emulsion until used

80 Hartley guinea pigs weighing 200-250g with half males and half females were selected in this study, the Hartley guinea pigs were qualified in an adaptive breeding observation. 10 guinea pigs (half male and half female) were used as a normal control group based on a random allocation, and no drugs were administrated to them; the other 70 guinea pigs (half males and half females) were treated with 5% propranolol hydrochloride emulsion (about 200 µL) evenly in the skins behind both of ears, 2 times a day, continuing treating 4 weeks, for modeling. 4 weeks later, 10 guinea pigs were sacrificed to examine whether the model was successful (with the thickness of the ear skin as an index). The remaining 60 guinea pigs were then layered with Excel according to weights, 6 guinea pigs every layer, and the guinea pigs in each layer were randomly assigned to each group, 10 guinea pigs every group, and the guinea pigs were divided into 6 groups in total, that is the model control group, the group of the present invention, the group of patent 201210183904.3, the group of the patent of 200910091066.5, the group of methotrexate tablet and the group of yujin yinxie tablet. The guinea pigs in each group were given by intragastric administration at a volume of 20 mL·kg⁻¹·d⁻¹ in the morning, once every day for 10 days. The normal control group and the model control group were given equal volume of pure water. The guinea pigs were sacrificed one hour after the last administration, and the skin behind both of ears was took off and fixed with 10% formaldehyde solution, embedded in paraffin, and stained with HE, observed under an optical microscope. 10 high-power fields were counted, the thickness of the epidermis was measured with a micrometer, and averaged; the auricular pathology of guinea pigs was used to perform a histological examination, observe vasodilation situations, which were denoted as 0, 0.5, 1 and 2 points according to normal, light, medium and heavy degrees.

Results: The histopathological examination showed that the normal control group showed thinner stratum corneum, flat trochanterellus and unobvious telangiectasia; the model control group showed extensive hyperkeratosis and focal parakeratosis, thinning or disappearance of granule layer, obvious thickening of the spinous layer, trochanterellus showed wave-like ups and downs, dermis superficial telangiectasia and congestion. Data showed that compared with the normal control group, the model control group significantly increased the thickness of the epidermis of guinea pig ears and dilate blood vessels, the difference was significant (P <0.01), indicating that the psoriasis-like model of guinea pig is success.

The results of histopathological examination showed that the group of the present invention, the group of patent 201210183904.3, the group of patent 200910091066.5, the group of methotrexate tablet and the group of yujin yinxie tablet showed the phenomenon of hyperkeratosis and parakeratosis was obviously alleviated, the spinous layer was thinned obviously, and a small amount of telangiectasia was found in the dermis. The data showed that the group of the present invention, the group of patent 200910103904.3, the group of patent 200910091066.5, the group of methotrexate tablet and the group of yujin yinxie tablet could significantly reduce the thickness of the epidermis of guinea pig ears and reduce hemangiectasis *(P* <0.01). The group of methotrexate tablet and the group of yujin yinxie tablet could significantly reduce the thickness of the epidermis of guinea pig ears and hemangiectasis, the difference was significant (P <0.01) compared with the model control group. It is indicated that the present invention has an inhibitory effect on psoriasis-like pathological changes in the ears of guinea pigs. The results are shown in Table 5.

**Table 5 Effect of the drugs of the present invention on propranolol-induced guinea pig psoriasis model (x̅ ± s n = 10)**

| Group | Dosage (g crude drug·kg⁻¹·d⁻¹) | Epidermal thickness (µm) | Blood vessel dilatation (score) |
|---|---|---|---|
| Normal control group | | 87.72±9.38 | 0 |
| model control group | | 148.04±11.05^{△△} | 1.70 ± 0.19^{△△} |
| Group of the present invention | 5.26 | 85.6±6.00^{∗∗} | 0.35±0.17^{∗∗} |
| Group of 201210183904.3 | 10.81 | 110.98±5.37^{∗∗} | 0.78±0.08^{∗∗} |
| Group of 200910091066.5 | 12.76 | 100.56±4.51 ^{∗∗} | 0.65±0.08^{∗∗} |
| Group of methotrexate tablet | 1.88 mg | 96.61±4.78^{∗∗} | 0.42±0.12^{∗∗} |
| Group of yujin yinxie tablet | 0.80 | 95.09±4.65^{∗∗} | 0.38±0.11 ^{∗∗} |

| | | | |
|---|---|---|---|
| Note: Comparing the model control group with the normal control group, ^{△}P<0.05, ^{△△}P <0.01; comparing treated group with model control group, ^{∗∗}P <0.01. | | | |

As can be seen from the above table, the group of the present invention is slightly superior to the positive control group, is remarkable superior to the group of patent 201210183904.3 and the group of patent 200910091066.5.

### EXAMPLE 6

Toxicity comparison among the formulations:
(1) The experimental mice were adaptive fed for 3 days, animals were fasted except water for 16 hours before the experiment. The experimental mice were divided into four groups, i.e. blank control group, the group of patent 200910091066.5, the group of patent 201210183904. 3 and the group of the present invention, by random number table method, 20 each group, half males and half females.
(2) The normal saline was administered to the blank control group. The other three groups were administered according to the maximum tolerable dosage (the maximum dosage of the intragastric administration), which was converted to human clinical dosage, i.e. 2 times a day, only gavage 1 day.
(3) The state of the mice was observed after each administration, and the number of dead mice was counted.
(4) For the mice that did not die after 48 hours, the state of 2 weeks was observed.

### Experimental results

The maximum dosage of group of patent 200910091066.5 was 79 times of that of the clinical group, and the dosage of first intragastric administration was the maximum dosage (79 times). After the intragastric administration, the mice were feathered, rounded, the activities of the mice were reduced, some of mice breathed heavier. In 15min, 9 mice died, 3 males and 6 females. Mice were breathing heavier, jumping, nose and mouth cyanosed before death, and show dark purple between claws after death.

After 4 hours, the survived mice returned to normal.

The dosage of second intragastric administration was accumulated to 158 times of that of the clinical group. In 20 minutes after the intragastric administration, 5 mice died. After 24 hours, 5 mice died.

The maximum dosage of group of patent 201210183904. 3 was 92 times of that of the clinical group. After the first administration, the mice did not die, but had symptoms of feathering, reduced activity, rounded, breathing heavier. After the second administration, 3 mice died.

The maximum dosage of the group of the present invention was 195 times of the clinical dosage. After the first administration, the mice had symptoms of fatigue, reduced activity and no death. After the second administration, the mice show the same effect as the first administration, and had no other discomfort in addition to reduced activity. The results are shown in Table 6.

**Table 6 Comparison of Toxicity of the drugs of the present invention with the drugs of the patents 2009. and 2012.**

| Observation indexs | Group of 200910091066.5 | Group of 201210183904.3 | Group of the present invention |
|---|---|---|---|
| Dosage of administration (equivalent to multiples of clinical dosage) | 158 | 184 | 390 |
| The percentage of dead animals | 95% | 15% | 0 |

## Claims

1. A traditional Chinese medicine composition, **characterized in that** the composition is prepared from materials in the following parts by weight:
5-15 parts of paeoniae rasix rubia (red peony root), 3-9 parts of curcumae rhizoma (rhizoma zedoariae), 10-20 parts of sarcandrae herba (sarcandra glabra), 10-20 parts of smilacis glabrae rhizoma (glabrous greenbrier rhizome), and 5-15 parts of mume fructus (black plum).

2. A traditional Chinese medicine composition, **characterized in that** the composition is prepared from materials in the following parts by weight:
9 parts of paeoniae radix rubra (Red peony root), 6 parts of curcumae rhizoma (rhizoma zedoariae), 15 parts of sarcandrae herba (sarcandra glabra, Glabrous Sarcandra Herb), 15 parts of samilacis glabrae rhizoma (soil glabrous greenbrier rhizome), and 6 parts of muma fructus (black plum).

3. A traditional Chinese medicine composition for use in treating psoriasis, **characterized in that** the traditional Chinese medicine composition is prepared from materials in the following parts by weight:
5-15 parts of paeoniae radix rubra (red peony root), 3-9 parts of curcumae rhizoma (rhizoma zedoariae), 10-20 parts of sarcandrae herba (sarcandra glabra), 10-20 parts of samilacis glabrae rhizoma (glabrous greenbrier rhizome), and 5-15 parts of muma fructus (black plum).

4. A traditional Chinese medicine composition for use in treating psoriasis, **characterized in that** the traditional Chinese medicine composition is prepared from materials in the following parts by weight:
9 parts of paeoniae radix rubra (Red peony root), 6 parts of curcumae rhizoma (rhizoma zedoariae), 15 parts of sarcandrae herba (sarcandra glabra, Glabrous Sarcandra Herb), 15 parts of samilacis glabrae rhizoma (soil glabrous greenbrier rhizome), and 6 parts of muma fructus (black plum).

5. The traditional Chinese medicine composition for use according toclaim 3, wherein the traditional Chinese medicine composition is administered in a medicament, **characterized in that** the medicament is administered to patients in need of treatment by oral administration, injection administration, sublingual administration, rectal administration, vaginal administration, transdermal administration, or spray inhalation route.

6. The traditional Chinese medicine composition for use according toclaim 3, wherein the traditional Chinese medicine composition is administered in a medicament, **characterized in that** the medicament comprises a therapeutically effective amount of the traditional Chinese medicine composition and a pharmaceutically acceptable carrier.

7. The traditional Chinese medicine composition for use according toclaim 6, wherein the traditional Chinese medicine composition is administered in a medicament, **characterized in that** the medicament is in the forms of tablets, granules, capsules, pills, dropping pills, powders, electuary, syrups, emplastrum, mixtures, medicated liquor, tinctures, lozenges, liquid extract and extract, plasters, gels, ointments, tea agents, lotions, coating agents, liniments, aerosols or sprays.

8. The traditional Chinese medicine composition for use according toclaim 4, wherein the traditional Chinese medicine composition is administered in a medicament, **characterized in that** the medicament is administered to patients in need of treatment by oral administration, injection administration, sublingual administration, rectal administration, vaginal administration, transdermal administration, or spray inhalation route.

9. The traditional Chinese medicine composition for use according toclaim 4, wherein the traditional Chinese medicine composition is administered in a medicament, **characterized in that** the medicament comprises a therapeutically effective amount of the traditional Chinese medicine composition and a pharmaceutically acceptable carrier.

10. A traditional Chinese medicine composition for use in a method for treating psoriasis in a subject, wherein the traditional Chinese medicine composition is administered to the subject in a pharmaceutically effective amount, wherein the traditional Chinese medicine composition is prepared from materials in the following parts by weight:
5-15 parts of paeoniae rasix rubia (red peony root), 3-9 parts of curcumae rhizoma (rhizoma zedoariae), 10-20 parts of sarcandrae herba (sarcandra glabra), 10-20 parts of smilacis glabrae rhizoma (glabrous greenbrier rhizome), and 5-15 parts of mume fructus (black plum).

11. The traditional Chinese medicine composition for use according to claim 10, wherein the traditional Chinese medicine composition is administered to the subject by oral administration, injection administration, sublingual administration, rectal administration, vaginal administration, transdermal administration, or spray inhalation route.

12. A traditional Chinese medicine composition for use in a method for treating psoriasis in a subject, wherein the traditional Chinese medicine composition is administered to the subject in a pharmaceutically effective amount, wherein the traditional Chinese medicine composition is prepared from materials in the following parts by weight:
9 parts of paeoniae radix rubra (Red peony root), 6 parts of curcumae rhizoma (rhizoma zedoariae), 15 parts of sarcandrae herba (sarcandra glabra, Glabrous Sarcandra Herb), 15 parts of samilacis glabrae rhizoma (soil glabrous greenbrier rhizome), and 6 parts of muma fructus (black plum).

13. The traditional Chinese medicine composition for use according to claim 12, wherein the traditional Chinese medicine composition is administered to the subject by oral administration, injection administration, sublingual administration, rectal administration, vaginal administration, transdermal administration, orspray inhalation route.

14. A traditional Chinese medicine composition for use in a method for treating psoriasis in a subject, wherein the traditional Chinese medicine composition is administered to the subject in a medicament comprising a pharmaceutically effective amount of the traditional Chinese medicine composition and a pharmaceutically acceptable carrier, wherein the traditional Chinese medicine composition is prepared from materials in the following parts by weight:
5-15 parts of paeoniae rasix rubia (red peony root), 3-9 parts of curcumae rhizoma (rhizoma zedoariae), 10-20 parts of sarcandrae herba (sarcandra glabra), 10-20 parts of smilacis glabrae rhizoma (glabrous greenbrier rhizome), and 5-15 parts of mume fructus (black plum).

15. The traditional Chinese medicine composition for use according to claim 14, wherein the medicament is administered to the subject by oral administration, injection administration, sublingual administration, rectal administration, vaginal administration, transdermal administration, or spray inhalation route.

16. The traditional Chinese medicine composition for use according to claim 14, wherein the medicament is in the forms of tablets, granules, capsules, pills, dropping pills, powders, electuary, syrups, emplastrum, mixtures, medicated liquor, tinctures, lozenges, liquid extract and extract, plasters, gels, ointments, tea agents, lotions, coating agents, liniments, aerosols or sprays.

17. A traditional Chinese medicine composition for use in a method for treating psoriasis in a subject, wherein the traditional Chinese medicine composition is administered to the subject in a medicament comprising a pharmaceutically effective amount of the traditional Chinese medicine composition and a pharmaceutically acceptable carrier, wherein the traditional Chinese medicine composition is prepared from materials in the following parts by weight:
9 parts of paeoniae radix rubra (Red peony root), 6 parts of curcumae rhizoma (rhizoma zedoariae), 15 parts ofsarcandrae herba (sarcandra glabra, Glabrous Sarcandra Herb), 15 parts of samilacis glabrae rhizoma (soil glabrous greenbrier rhizome), and 6 parts of muma fructus (black plum).

18. The traditional Chinese medicine composition for use according to claim 17, wherein the medicament is administered to the subject by oral administration, injection administration, sublingual administration, rectal administration, vaginal administration, transdermal administration, or spray inhalation route.

19. The traditional Chinese medicine composition for use according to claim 17, wherein the medicament is in the forms of tablets, granules, capsules, pills, dropping pills, powders, electuary, syrups, emplastrum, mixtures, medicated liquor, tinctures, lozenges, liquid extract and extract, plasters, gels, ointments, tea agents, lotions, coating agents, liniments, aerosols or sprays.

## Patentansprüche

1. Zusammensetzung der traditionellen chinesischen Medizin, **dadurch gekennzeichnet, dass** die Zusammensetzung aus Materialien in den folgenden Gewichtsteilen hergestellt wird:
5-15 Teile Paeoniae rasix rubia (rote Pfingstrosenwurzel), 3-9 Teile Curcumae rhizoma (Rhizoma zedoariae), 10-20 Teile Sarcandrae herba (Sarcandra glabra), 10-20 Teile Smilacis glabrae rhizoma (Rhizom der kahlen Sarsaparille) und 5-15 Teile Mume fructus (schwarze Pflaume).

2. Zusammensetzung der traditionellen chinesischen Medizin, **dadurch gekennzeichnet, dass** die Zusammensetzung aus Materialien in den folgenden Gewichtsteilen hergestellt wird:
9 Teile Paeoniae radix rubia (rote Pfingstrosenwurzel), 6 Teile Curcumae rhizoma (Rhizoma zedoariae), 15 Teile Sarcandrae herba (Sarcandra glabra, kahles Sarcandra-Kraut), 15 Teile Smilacis glabrae rhizoma (Rhizom der kahlen Boden-Sarsaparille) und 6 Teile Muma fructus (schwarze Pflaume).

3. Zusammensetzung der traditionellen chinesischen Medizin zur Verwendung bei einer Behandlung von Psoriaris, **dadurch gekennzeichnet, dass** die Zusammensetzung der traditionellen chinesischen Medizin aus Materialien in den folgenden Gewichtsteilen hergestellt wird:
5-15 Teile Paeoniae radix rubra (rote Pfingstrosenwurzel), 3-9 Teile Curcumae rhizoma (Rhizoma zedoariae), 10-20 Teile Sarcandrae herba (Sarcandra glabra), 10-20 Teile Samilacis glabrae rhizoma (Rhizom der kahlen Sarsaparille) und 5-15 Teile Muma fructus (schwarze Pflaume).

4. Zusammensetzung der traditionellen chinesischen Medizin zur Verwendung bei einer Behandlung von Psoriaris, **dadurch gekennzeichnet, dass** die Zusammensetzung der traditionellen chinesischen Medizin aus Materialien in den folgenden Gewichtsteilen hergestellt wird:
9 Teile Paeoniae radix rubia (rote Pfingstrosenwurzel), 6 Teile Curcumae rhizoma (Rhizome zedoariae), 15 Teile Sarcandrae herba (Sarcandra glabra, kahles Sarcandra-Kraut), 15 Teile Smilacis glabrae rhizoma (Rhizom der kahlen Boden-Sarsaparille) und 6 Teile Muma fructus (schwarze Pflaume).

5. Zusammensetzung der traditionellen chinesischen Medizin zur Verwendung nach Anspruch 3, wobei die Zusammensetzung der traditionellen chinesischen Medizin in einem Arzneimittel verabreicht wird, **dadurch gekennzeichnet, dass** das Arzneimittel behandlungsbedürftigen Patienten durch orale Verabreichung, Injektionsverabreichung, sublinguale Verabreichung, rektale Verabreichung, vaginale Verabreichung, transdermale Verabreichung oder auf dem Sprayinhalationsweg verabreicht wird.

6. Zusammensetzung der traditionellen chinesischen Medizin zur Verwendung nach Anspruch 3, wobei die Zusammensetzung der traditionellen chinesischen Medizin in einem Arzneimittel verabreicht wird, **dadurch gekennzeichnet, dass** das Arzneimittel eine therapeutisch wirksame Menge der Zusammensetzung der traditionellen chinesischen Medizin und einen pharmazeutisch unbedenklichen Träger umfasst.

7. Zusammensetzung der traditionellen chinesischen Medizin zur Verwendung nach Anspruch 6, wobei die Zusammensetzung der traditionellen chinesischen Medizin in einem Arzneimittel verabreicht wird, **dadurch gekennzeichnet, dass** das Arzneimittel in Form von Tabletten, Granalien, Kapseln, Pillen, Tropfpillen, Pulvern, Electuarium, Sirupen, Pflastern, Mischungen, medizinischen Likören, Tinkturen, Pastillen, flüssigem Extrakt und Extrakt, Heftpflastern, Gelen, Salben, Teemitteln, Lotionen, Überzugsmitteln, Linimenten, Aerosolen oder Sprays ist.

8. Zusammensetzung der traditionellen chinesischen Medizin zur Verwendung nach Anspruch 4, wobei die Zusammensetzung der traditionellen chinesischen Medizin in einem Arzneimittel verabreicht wird, **dadurch gekennzeichnet, dass** das Arzneimittel behandlungsbedürftigen Patienten durch orale Verabreichung, Injektionsverabreichung, sublinguale Verabreichung, rektale Verabreichung, vaginale Verabreichung, transdermale Verabreichung oder auf dem Sprayinhalationsweg verabreicht wird.

9. Zusammensetzung der traditionellen chinesischen Medizin zur Verwendung nach Anspruch 4, wobei die Zusammensetzung der traditionellen chinesischen Medizin in einem Arzneimittel verabreicht wird, **dadurch gekennzeichnet, dass** das Arzneimittel eine therapeutisch wirksame Menge der Zusammensetzung der traditionellen chinesischen Medizin und einen pharmazeutisch unbedenklichen Träger umfasst.

10. Zusammensetzung der traditionellen chinesischen Medizin zur Verwendung in einem Verfahren zum Behandeln von Psoriaris in einem Subjekt, wobei die Zusammensetzung der traditionellen chinesischen Medizin in einer pharmazeutisch wirksamen Menge an das Subjekt verabreicht wird, wobei die Zusammensetzung der traditionellen chinesischen Medizin aus Materialien in den folgenden Gewichtsteilen hergestellt wird:
5-15 Teile Paeoniae rasix rubia (rote Pfingstrosenwurzel), 3-9 Teile Curcumae rhizoma (Rhizoma zedoariae), 10-20 Teile Sarcandrae herba (Sarcandra glabra), 10-20 Teile Smilacis glabrae rhizoma (Rhizom der kahlen Sarsaparille) und 5-15 Teile Mume fructus (schwarze Pflaume).

11. Zusammensetzung der traditionellen chinesischen Medizin zur Verwendung nach Anspruch 10, wobei die Zusammensetzung der traditionellen chinesischen Medizin dem Subjekt durch orale Verabreichung, Injektionsverabreichung, sublinguale Verabreichung, rektale Verabreichung, vaginale Verabreichung, transdermale Verabreichung oder auf dem Sprayinhalationsweg verabreicht wird.

12. Zusammensetzung der traditionellen chinesischen Medizin zur Verwendung in einem Verfahren zum Behandeln von Psoriaris in einem Subjekt, wobei die Zusammensetzung der traditionellen chinesischen Medizin in einer pharmazeutisch wirksamen Menge an das Subjekt verabreicht wird, wobei die Zusammensetzung der traditionellen chinesischen Medizin aus Materialien in den folgenden Gewichtsteilen hergestellt wird:
9 Teile Paeoniae radix rubia (rote Pfingstrosenwurzel), 6 Teile Curcumae rhizoma (Rhizoma zedoariae), 15 Teile Sarcandrae herba (Sarcandra glabra, kahles Sarcandra-Kraut), 15 Teile Smilacis glabrae rhizoma (Rhizom der kahlen Boden-Sarsaparille) und 6 Teile Muma fructus (schwarze Pflaume).

13. Zusammensetzung der traditionellen chinesischen Medizin zur Verwendung nach Anspruch 12, wobei die Zusammensetzung der traditionellen chinesischen Medizin dem Subjekt durch orale Verabreichung, Injektionsverabreichung, sublinguale Verabreichung, rektale Verabreichung, vaginale Verabreichung, transdermale Verabreichung oder auf dem Sprayinhalationsweg verabreicht wird.

14. Zusammensetzung der traditionellen chinesischen Medizin zur Verwendung in einem Verfahren zum Behandeln von Psoriaris in einem Subjekt, wobei die Zusammensetzung der traditionellen chinesischen Medizin dem Subjekt in einem Arzneimittel verabreicht wird, das eine pharmazeutisch wirksame der Zusammensetzung der traditionellen chinesischen Medizin und einen pharmazeutisch unbedenklichen Träger umfasst, wobei die Zusammensetzung der traditionellen chinesischen Medizin aus Materialien in den folgenden Gewichtsteilen hergestellt wird:
5-15 Teile Paeoniae rasix rubia (rote Pfingstrosenwurzel), 3-9 Teile Curcumae rhizoma (Rhizoma zedoariae), 10-20 Teile Sarcandrae herba (Sarcandra glabra), 10-20 Teile Smilacis glabrae rhizoma (Rhizom der kahlen Sarsaparille) und 5-15 Teile Mume fructus (schwarze Pflaume).

15. Zusammensetzung der traditionellen chinesischen Medizin zur Verwendung nach Anspruch 14, wobei das Arzneimittel dem Subjekt durch orale Verabreichung, Injektionsverabreichung, sublinguale Verabreichung, rektale Verabreichung, vaginale Verabreichung, transdermale Verabreichung oder auf dem Sprayinhalationsweg verabreicht wird.

16. Zusammensetzung der traditionellen chinesischen Medizin zur Verwendung nach Anspruch 14, wobei das Arzneimittel in Form von Tabletten, Granalien, Kapseln, Pillen, Tropfpillen, Pulvern, Electuarium, Sirupen, Pflastern, Mischungen, medizinischen Likören, Tinkturen, Pastillen, flüssigem Extrakt und Extrakt, Heftpflastern, Gelen, Salben, Teemitteln, Lotionen, Überzugsmitteln, Linimenten, Aerosolen oder Sprays ist.

17. Zusammensetzung der traditionellen chinesischen Medizin zur Verwendung in einem Verfahren zum Behandeln von Psoriaris in einem Subjekt, wobei die Zusammensetzung der traditionellen chinesischen Medizin dem Subjekt in einem Arzneimittel verabreicht wird, das eine pharmazeutisch wirksame der Zusammensetzung der traditionellen chinesischen Medizin und einen pharmazeutisch unbedenklichen Träger umfasst, wobei die Zusammensetzung der traditionellen chinesischen Medizin aus Materialien in den folgenden Gewichtsteilen hergestellt wird:
9 Teile Paeoniae radix rubia (rote Pfingstrosenwurzel), 6 Teile Curcumae rhizoma (Rhizoma zedoariae), 15 Teile Sarcandrae herba (Sarcandra glabra, kahles Sarcandra-Kraut), 15 Teile Smilacis glabrae rhizoma (Rhizom der kahlen Boden-Sarsaparille) und 6 Teile Muma fructus (schwarze Pflaume).

18. Zusammensetzung der traditionellen chinesischen Medizin zur Verwendung nach Anspruch 17, wobei das Arzneimittel dem Subjekt durch orale Verabreichung, Injektionsverabreichung, sublinguale Verabreichung, rektale Verabreichung, vaginale Verabreichung, transdermale Verabreichung oder auf dem Sprayinhalationsweg verabreicht wird.

19. Zusammensetzung der traditionellen chinesischen Medizin zur Verwendung nach Anspruch 17, wobei das Arzneimittel in Form von Tabletten, Granalien, Kapseln, Pillen, Tropfpillen, Pulvern, Electuarium, Sirupen, Pflastern, Mischungen, medizinischen Likören, Tinkturen, Pastillen, flüssigem Extrakt und Extrakt, Heftpflastern, Gelen, Salben, Teemitteln, Lotionen, Überzugsmitteln, Linimenten, Aerosolen oder Sprays ist.

## Revendications

1. Composition de médecine traditionnelle chinoise, **caractérisée en ce que** la composition est préparée à partir de matières dans les parties en poids suivantes :
5 à 15 parties de paeoniae rasix rubia (racine de pivoine rouge), 3 à 9 parties de curcumae rhizoma (zédoaire rhizome), 10 à 20 parties de sarcandrae herba (sarcandra glabra), 10 à 20 parties de smilacis glabrae rhizoma (rhizome glabre de salsepareille) et 5 à 15 parties de mume fructus (prune noire).

2. Composition de médecine traditionnelle chinoise, **caractérisée en ce que** la composition est préparée à partir de matières dans les parties en poids suivantes :
9 parties de paeoniae radix rubra (racine de pivoine rouge), 6 parties de curcumae rhizoma (zédoaire rhizome), 15 parties de sarcandrae herba (sarcandra glabra, herbe de sarcandra glabre), 15 parties de samilacis glabrae rhizoma (rhizome glabre de salsepareille en sol) et 6 parties de muma fructus (prune noire).

3. Composition de médecine traditionnelle chinoise destinée à être utilisée dans le traitement du psoriasis, **caractérisée en ce que** la composition de médecine traditionnelle chinoise est préparée à partir de matières dans les parties en poids suivantes :
5 à 15 parties de paeoniae radix rubra (racine de pivoine rouge), 3 à 9 parties de curcumae rhizome (zédoaire rhizome), 10 à 20 parties de sarcandrae herba (sarcandra glabra), 10 à 20 parties de samilacis glabrae rhizoma (rhizome glabre de salsepareille) et 5 à 15 parties de muma fructus (prune noire).

4. Composition de médecine traditionnelle chinoise destinée à être utilisée dans le traitement du psoriasis, **caractérisée en ce que** la composition de médecine traditionnelle chinoise est préparée à partir de matières dans les parties en poids suivantes :
9 parties de paeoniae radix rubra (racine de pivoine rouge), 6 parties de curcumae rhizome (zédoaire rhizome), 15 parties de sarcandrae herba (sarcandra glabra, herbe de sarcandra glabre), 15 parties de samilacis glabrae rhizoma (rhizome glabre de salsepareille en sol) et 6 parties de muma fructus (prune noire).

5. Composition de médecine traditionnelle chinoise à utiliser selon la revendication 3, dans laquelle la composition de médecine traditionnelle chinoise est administrée dans un médicament, **caractérisée en ce que** le médicament est administré à des patients nécessitant un traitement par administration orale, administration par injection, administration sublinguale, administration rectale, administration vaginale, administration transdermique ou voie d'inhalation par pulvérisation.

6. Composition de médecine traditionnelle chinoise à utiliser selon la revendication 3, dans laquelle la composition de médecine traditionnelle chinoise est administrée dans un médicament, **caractérisée en ce que** le médicament comprend une quantité thérapeutiquement efficace de la composition de médecine traditionnelle chinoise et un support pharmaceutiquement acceptable.

7. Composition de médecine traditionnelle chinoise à utiliser selon la revendication 6, dans laquelle la composition de médecine traditionnelle chinoise est administrée dans un médicament, **caractérisée en ce que** le médicament est sous forme de comprimés, granulés, capsules, pilules, pillules linguales, poudres, électuaire, sirops, emplastrum, mélanges, liqueurs médicamenteuses, teintures, pastilles, extraits et extraits liquides, emplâtres, gels, onguents, agents de thé, lotions, agents d'enrobage, liniments, aérosols ou pulvérisateurs.

8. Composition de médecine traditionnelle chinoise à utiliser selon la revendication 4, dans laquelle la composition de médecine traditionnelle chinoise est administrée dans un médicament, **caractérisée en ce que** le médicament est administré à des patients nécessitant un traitement par administration orale, administration par injection, administration sublinguale, administration rectale, administration vaginale, administration transdermique ou voie d'inhalation par pulvérisation.

9. Composition de médecine traditionnelle chinoise à utiliser selon la revendication 4, dans laquelle la composition de médecine traditionnelle chinoise est administrée dans un médicament, **caractérisée en ce que** le médicament comprend une quantité thérapeutiquement efficace de la composition de médecine traditionnelle chinoise et un support pharmaceutiquement acceptable.

10. Composition de médecine traditionnelle chinoise destinée à être utilisée dans un procédé de traitement du psoriasis chez un sujet, dans laquelle la composition de médecine traditionnelle chinoise est administrée au sujet en une quantité pharmaceutiquement efficace, dans laquelle la composition de médecine traditionnelle chinoise est préparée à partir de matières dans les parties en poids suivantes :
5 à 15 parties de paeoniae rasix rubia (racine de pivoine rouge), 3 à 9 parties de curcumae rhizoma (zédoaire rhizome), 10 à 20 parties de sarcandrae herba (sarcandra glabra), 10 à 20 parties de smilacis glabrae rhizoma (rhizome glabre de salsepareille) et 5 à 15 parties de mume fructus (prune noire).

11. Composition de médecine traditionnelle chinoise à utiliser selon la revendication 10, dans laquelle la composition de médecine traditionnelle chinoise est administrée au sujet par administration orale, administration par injection, administration sublinguale, administration rectale, administration vaginale, administration transdermique ou voie d'inhalation par pulvérisation.

12. Composition de médecine traditionnelle chinoise destinée à être utilisée dans un procédé de traitement du psoriasis chez un sujet, dans laquelle la composition de médecine traditionnelle chinoise est administrée au sujet en une quantité pharmaceutiquement efficace, dans laquelle la composition de médecine traditionnelle chinoise est préparée à partir de matières dans les parties en poids suivantes :
9 parties de paeoniae radix rubra (racine de pivoine rouge), 6 parties de curcumae rhizoma (zédoaire rhizome), 15 parties de sarcandrae herba (sarcandra glabra, herbe de sarcandra glabre), 15 parties de samilacis glabrae rhizoma (rhizome glabre de salsepareille en sol) et 6 parties de muma fructus (prune noire).

13. Composition de médecine traditionnelle chinoise à utiliser selon la revendication 12, dans laquelle la composition de médecine traditionnelle chinoise est administrée au sujet par administration orale, administration par injection, administration sublinguale, administration rectale, administration vaginale, administration transdermique ou voie d'inhalation par pulvérisation.

14. Composition de médecine traditionnelle chinoise destinée à être utilisée dans une méthode de traitement du psoriasis chez un sujet, dans laquelle la composition de médecine traditionnelle chinoise est administrée au sujet dans un médicament comprenant une quantité pharmaceutiquement efficace de la composition de médecine traditionnelle chinoise et un support pharmaceutiquement acceptable, dans laquelle la composition de médecine traditionnelle chinoise est préparée à partir de matières dans les parties en poids suivantes :
5 à 15 parties de paeoniae rasix rubia (racine de pivoine rouge), 3 à 9 parties de curcumae rhizoma (zédoaire rhizome), 10 à 20 parties de sarcandrae herba (sarcandra glabra), 10 à 20 parties de smilacis glabrae rhizoma (rhizome glabre de salsepareille) et 5 à 15 parties de mume fructus (prune noire).

15. Composition de médecine traditionnelle chinoise à utiliser selon la revendication 14, dans laquelle le médicament est administré au sujet par administration orale, administration par injection, administration sublinguale, administration rectale, administration vaginale, administration transdermique ou voie d'inhalation par pulvérisation.

16. Composition de médecine traditionnelle chinoise à utiliser selon la revendication 14, dans laquelle le médicament est sous forme de comprimés, granulés, gélules, pilules, pilules linguales, poudres, électuaires, sirops, emplastrum, mélanges, liqueurs médicamenteuses, teintures, pastilles, extraits liquides et extraits, emplâtres, gels, onguents, agents de thé, lotions, agents d'enrobage, liniments, aérosols ou pulvérisations.

17. Composition de médecine traditionnelle chinoise destinée à être utilisée dans une méthode de traitement du psoriasis chez un sujet, dans laquelle la composition de médecine traditionnelle chinoise est administrée au sujet dans un médicament comprenant une quantité pharmaceutiquement efficace de la composition de médecine traditionnelle chinoise et un support pharmaceutiquement acceptable, dans laquelle la composition de médecine traditionnelle chinoise est préparée à partir de matières dans les parties en poids suivantes :
9 parties de paeoniae radix rubra (racine de pivoine rouge), 6 parties de rhizome de curcuma (zédoaire rhizome), 15 parties de sarcandrae herba (sarcandra glabra, herbe de sarcanda glabre), 15 parties de samilacis glabrae rhizoma (rhizome glabre de salsepareille en sol) et 6 parties de muma fructus (prune noire).

18. Composition de médecine traditionnelle chinoise à utiliser selon la revendication 17, dans laquelle le médicament est administré au sujet par administration orale, administration par injection, administration sublinguale, administration rectale, administration vaginale, administration transdermique ou voie d'inhalation par pulvérisation.

19. Composition de médecine traditionnelle chinoise à utiliser selon la revendication 17, dans laquelle le médicament est sous forme de comprimés, granulés, capsules, pilules, pilules linguales, poudres, électuaires, sirops, emplastrum, mélanges, liqueurs médicamenteuses, teintures, pastilles, extraits liquides et extraits, emplâtres, gels, onguents, agents de thé, lotions, agents d'enrobage, liniments, aérosols ou pulvérisateurs.
